(19) Europäisches Patentamt
European Patent Office
Office européen des brevets

(11) EP 0 904 773 B1

(12) EUROPÄISCHE PATENTSCHRIFT

(45) Veröffentlichungstag und Bekanntmachung des Hinweises auf die Patenterteilung:
**10.03.2010 Patentblatt 2010/10**

(51) Int Cl.:
***A61K 8/39*** *(2006.01)* ***A61K 8/85*** *(2006.01)*
***A61K 8/06*** *(2006.01)* ***A61Q 19/00*** *(2006.01)*

(21) Anmeldenummer: **98118102.7**

(22) Anmeldetag: **24.09.1998**

# (54) Dünnflüssige kosmetische oder dermatologische Zubereitungen vom Typ Wasser-in-Öl

Low-viscosity water-in-oil cosmetic or dermatological composition

Composition cosmétique ou dermatologique du type eau-dans-huile à faible viscosité

(84) Benannte Vertragsstaaten:
**AT BE CH CY DE DK ES FI FR GB GR IE IT LI LU MC NL PT SE**

(30) Priorität: **26.09.1997 DE 19742467**

(43) Veröffentlichungstag der Anmeldung:
**31.03.1999 Patentblatt 1999/13**

(73) Patentinhaber: **Beiersdorf Aktiengesellschaft
20245 Hamburg (DE)**

(72) Erfinder:
- **Gers-Barlag, Heinrich, Dr.
25495 Kummerfeld (DE)**
- **von der Fecht, Stephanie
22869 Schenefeld (DE)**
- **Kröpke, Rainer
22527 Hamburg (DE)**
- **Schneider, Günther, Dr.
22607 Hamburg (DE)**

(56) Entgegenhaltungen:
**EP-A- 0 834 306 WO-A-98/17232
DE-A- 4 126 969 US-A- 5 162 378**

- **ADAM W E: "NEUE POLYALKYLENGLYKOL-COPOLYMERE FUER DIE KOSMETIK" SOFW-JOURNAL SEIFEN, OELE, FETTE, WACHSE, VERLAG FUR CHEMISCHE INDUSTRIE, H. ZIOLKOWSKY K.G. AUGSBURG, DE, Bd. 110, Nr. 15, 1984, Seiten 427-431, XP002057441 ISSN: 0942-7694**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 0 904 773 B1

## Beschreibung

Die vorliegende Erfindung betrifft kosmetische und dermatologische Zubereitungen vom Typ Wasser-in-Öl, Verfahren zu ihrer Herstellung sowie ihre Verwendung für kosmetische und medizinische Zwecke.

Die menschliche Haut übt als größtes Organ des Menschen zahlreiche lebenswichtige Funktionen aus. Mit durchschittlich etwa 2 $m^2$ Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

In bezug auf die Erhaltung von hauteigenen Substanzen, wie beispielsweise UV-Absorbern und Moisturizern, besitzen Kosmetika und Dermatika in Form von W/O-Formulierungen gegenüber O/W-Formulierungen Vorteile.

Natürlich ist dem Fachmann eine Vielzahl von Möglichkeiten bekannt, stabile W/O-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Der Stand der Technik kennt allerdings nur wenige Formulierungen, die so dünnflüssig sind, daß sie beispielsweise sprühbar wären.

Zudem haben dünnflüssige Zubereitungen des Standes der Technik häufig den Nachteil, daß sie instabil, auf einen engen Anwendungsbereich oder eine begrenzte Einsatzstoffauswahl begrenzt sind. Dünnflüssige Produkte, in denen beispielsweise stark polare Öle - wie die in handelsüblichen Produkten sonst häufig verwendeten Pflanzenöle - ausreichend stabilisiert sind, gibt es daher zur Zeit auf dem Markt nicht.

W/O-Emulsionen mit einer geringen Viskosität, die eine Lagerstabilität aufweisen, wie sie für marktgängige Produkte gefordert wird, sind nach dem Stand der Technik nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

Ein schwerwiegender Nachteil von Emulsionen des Standes der Technik ist oft deren mangelnde Stabilität gegenüber höheren Elektrolytkonzentrationen, was sich in einer Phasentrennung äußert. Es ist aber häufig wünschenswert, bestimmte Elektrolyte, wie beispielsweise wasserlösliche UV-Filter, einzusetzen, um deren sonstige physikalische, chemische bzw. physiologische Eigenschaften nutzen zu können. Zwar läßt sich in vielen Fällen durch geeignete Wahl des Emulgatorsystems in gewissem Maß Abhilfe schaffen, es treten dann aber ebensooft andere Nachteile auf.

Die angesprochenen Nachteile können beispielsweise darin liegen, daß Emulgatoren, wie letztendlich jede chemische Substanz, im Einzelfall allergische oder auf Überempfindlichkeit des Anwenders beruhende Reaktionen hervorrufen können, obwohl die Verwendung der üblichen kosmetischen Emulgatoren i.a. natürlich völlig unbedenklich ist.

Üblicherweise werden die Konzentrationen aller Bestandteile einer kosmetischen oder dermatologischen Zubereitungen in solchen Einheiten wie Gewichts-%, Mol-% etc. angegeben. Aufgrund ihrer mehr oder minder stark ausgeprägten Dissoziation in Kationen und Anionen, oftmals in mehreren Dissoziationsstufen, erscheint es im vorliegenden Fall hingegen zweckmäßiger von der Ionenstärke eines gegebenen Elektrolyten in seiner Lösung auszugehen.

Die Ionenstärke $I$ einer Elektrolytlösung ist definiert als

$$I = \frac{1}{2}\sum_i c_i z_i^2$$

wobei $c_i$ die Konzentrationen der einzelnen Ionensorten (in mol/l) und $z_i$ deren Ladungszahlen darstellen. Die physikalische Einheit der Ionenstärke ist die einer Konzentration (mol/l).

Eine 1-%ige (d.h. 0,17-molare) Kochsalzlösung beispielsweise hat dementsprechend eine Ionenstärke von $I$ = 0,17 mol/l.

In der Literatur (z.B. Seife, Öle, Fette, Wachse, Band 110 (15), S. 427 ff.) werden kosmetische Zubereitungen vom Typ Wasser in Öl, enthaltend eine Ölphase, eine Wasserphase mit Ionenstärke 1,7 und ein Emulgatorsystem beschrieben.

US 5162378 offenbart kosmetische Zubereitungen vom Typ Wasser in Öl, die in der Wasserphase 8 - 20% Elektrolyte enthalten und das Emulgatorsystem einen Ester von Polyglycerol und Fettsäure umfasst.

WO 9817232 offenbart eine kosmetische Zubereitung vom Typ Wasser in Öl, die ein Gemisch aus mehreren W/O Emulgatoren umfasst und die Wasserphase organische Salze beinhalten.

EP 834306 beschreibt kosmetische Zubereitungen vom Typ Wasser in Öl, die in der Wasserphase mindestens 2 Gew.% Elektrolyte enthalten und das Emulgatorsystem einen Ester von Polyglycerol und Fettsäure umfasst.

DE 4126969 beschreibt kosmetische Zubereitungen vom Typ Wasser in Öl, die in der Wasserphase mindestens 2 Gew.% Elektrolyte enthalten und das Emulgatorsystem Cetyl Dimethicone Copolyol, Polyglyceryl-4 Isostearat und Hexyllaurat umfasst.

DE 4243119 beschreibt kosmetische Zubereitungen vom Typ Wasser in Öl, die in eine Emulgatorkombination aus drei Komponenten umfassend Polyglyceryl-3 Diisostearat umfassen.

DE 4420516 offenbart die Verwendung von Polyglycerinpolyhydroxystearaten als W/O Emulgatoren für kosmetische Zubereitungen.

Eine Aufgabe der vorliegenden Erfindung war es, Zubereitungen vom Typ Wasser-in-Öl herzustellen, welche eine sehr geringe Viskosität haben und nicht die Nachteile des Standes der Technik aufweisen. Eine weitere Aufgabe der Erfindung war, Lösungswege zu kosmetischen oder dermatologischen, möglichst dünnflüssigen W/O-Emulsionen aufzudecken, welche gegenüber erhöhten Elektrolytkonzentrationen - bzw. erhöhten Ionenstärken - stabil sind.

Erstaunlicherweise werden diese Aufgaben gelöst durch dünnflüssige kosmetische oder dermatologische Zubereitungen vom Typ Wasser-in-Öl, enthaltend

(1) eine Ölphase, enthaltend ein oder mehrere Öle, Fette und/oder Wachse,
(2) eine Wasserphase, wobei die Wasserphase einen oder mehrere Elektrolyte gelöst enthält, wobei die Ionenstärke der wäßrigen Phase mindestens 0,075 mol/l beträgt und
(3) ein Emulgatorsystem, bestehend aus mindestens zwei Emulgatoren, welche gewählt werden aus der Gruppe Polyglyceryl-3-Diisostearat, Polyglyceryl-2-Polyhydroxystearat, PEG-30-Dipolyhydroxystearat sowie gewünschtenfalls enthaltend übliche kosmetische oder dermatologische Hilfs-, Zusatz- und/oder Wirkstoffe.

Die Viskosität der Zubereitungen ist kleiner als 2.000 mPa·s.

Es war für den Fachmann nicht vorauszusehen gewesen, dass die erfindungsgemäßen Zubereitungen

- einfacher zu formulieren sein,
- eine höhere Stabilität gegenüber einem Zerfall in Öl- und Wasserphasen und
- bessere sensorische Eigenschaften, wie beispielsweise die Verteilbarkeit auf der Haut oder das Einzugsvermögen in die Haut, aufweisen würden

als die Zubereitungen des Standes der Technik.

Die erfindungsgemäßen Zubereitungen stellen daher eine Bereicherung des Standes der Technik in bezug auf elektrolytstabile, dünnflüssige W/O-Emulsionen dar.

Zwar beschreiben sowohl die Deutsche Patentschrift 44 09 569 als auch die Deutsche Offenlegungsschrift 44 20 516 neue W/O-Emulgatoren in Form von Polyglycerinpolyricinoleaten bzw. Polyolpolyhydroxystearaten, welche beide gleichermaßen mit verschiedenen Ölen stabile niedrigviskose Emulsionen ergeben. Allerdings geht aus den Beispielen a.a.O. hervor, daß mit "niedrigviskos" Formulierungen gemeint sind, die eine Viskosität von minimal 8.000 mPa·s aufweisen. In der Regel liegt die Viskosität in den hier aufgeführten Beispielen sogar noch deutlich über einem Wert von 10.000 mPa·s.

Auch in Hinblick auf die Einarbeitung von Elektrolyten weist der Stand der Technik gemäß den beiden Schriften DE 44 09 569 C1 und DE 44 20 516 A1 nicht in Richtung auf die vorliegende Erfindung. Nach den Beschreibungen und den Beispielen können diese Zubereitungen wohl auch Salze enthalten. Allerdings ist a.a.O. als zu verwendender Elektrolyt nur Magnesiumsulfat offenbart.

Emulgatoren

Als besonders vorteilhaft haben sich erfindungsgemäße Emulgatorsysteme erwiesen, in welchen mindestens einer der beiden Emulgatoren Polyglyceryl-3-Diisostearat ist.

Solche Isostearinsäureester sind beispielsweise erhältlich unter der Produktbezeichnung "Lameform TGI" der Gesellschaft Henkel KGaA.

Als besonders vorteilhaft haben sich erfindungsgemäße Emulgatorsysteme erwiesen, in welchen mindestens einer der beiden Emulgatoren "Polyglyceryl-2-Polyhydroxystearat" ist welches unter den Registriernummern 156531-21-4 bzw. 144470-58-6 in den "Chemical Abstracts" abgelegt ist und welches beispielsweise unter der Warenbezeichnung DEHYMULS® PGPH von der Henkel KGaA erhältlich ist.

Es ist ganz besonders vorteilhaft im Sinne der vorliegenden Erfindung, wenn einer der beiden Emulgatoren PEG-30-Dipolyhydroxystearat ist, welches von der Gesellschaft ICI Surfactants unter der Warenbezeichnung ARLACEL® P135 verkauft wird.

Voraussetzung für die Verwendbarkeit der oben beschriebenen Emulgatoren für die erfindungsgemäßen Zwekke ist natürlich die kosmetische bzw. dermatologische Unbedenklichkeit der zugrundeliegenden Substanzen.

**Elektrolyte**

Erfindungsgemäß werden der oder die Elektrolyte vorteilhaft gewählt aus den folgenden Gruppen (1) bis (3):

(1) Bestimmte, zumeist als Alkalisalze vorliegende wasserlösliche UV-Filtersubstanzen, insbesondere solche, die an ihrem Molekülgerüst eine oder mehrere Sulfonsäuregruppen bzw. Sulfonatgruppen tragen; Beispiele hierfür sind: Die 2-Phenylbenzimidazol-5-sulfonsäure und ihre Salze, beispielsweise das Natrium-, Kalium- oder ihr Triethanolammonium-Salz

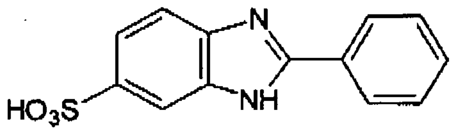

,

Sulfonsäure-Derivate von Benzophenonen, vorzugsweise 2-Hydroxy-4-methoxybenzophenon-5-sulfonsäure und ihre Salze, beispielsweise das entsprechende Natrium-, Kalium- oder Triethanolammonium-Salz:

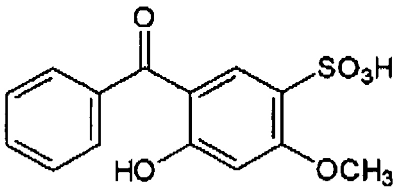

Sulfonsäure-Derivate des 3-Benzylidencamphers, wie z.B. 4-(2-Oxo-3-bornylidenmethyl)benzolsulfonsäure und ihre Salze, beispielsweise das entsprechende Natrium-, Kalium- oder Triethanolammonium-Salz:

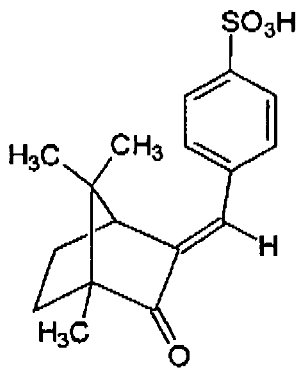

Die 2-Methyl-5-(2-oxo-3-bornylidenmethyl)benzolsulfonsäure und ihre Salze, beispielsweise das entsprechende Natrium-, Kalium- oder Triethanolammonium-Salz:

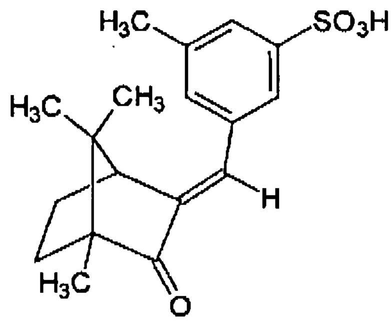

Das 1,4-di(2-oxo-10-sulfo-3-bornylidenmethyl-)Benzol und dessen Salze (die entsprechenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet:

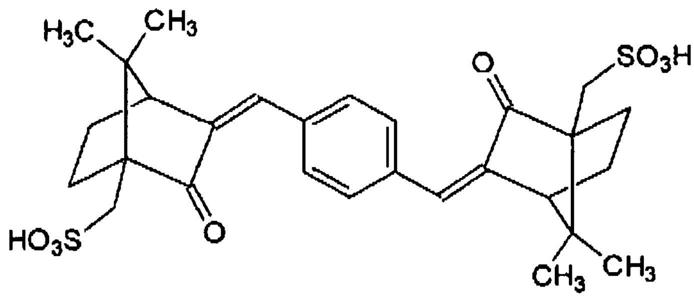

(2) Aminosäuren und deren Salze bzw. deren Anionen.

Aminosäuren sind Bestandteil des natürlichen Feuchtigkeitsfaktors (des sogenannten "Natural Moisturizing Factor"): Der Hydrolipidmantel ist die äußerste Schicht zur Regulierung des Feuchtigkeitsgehaltes der Hornschicht. Innerhalb der Hornschicht, d.h. von der lebenden Epidermis bis zur Oberfläche liegt ein starkes Feuchtigkeitsgefälle vor. Die wasserlösliche Bestandteile sind das als "Natural Moisturizing Factor" (NMF) bekannte Substanzgemisch, das hydrophil und schwach hygroskopisch ist und wesentliche Funktionen besitzt.

Der Zusatz von Aminosäuren, insbesondere essentieller Aminosäuren, ist daher als vorteilhaft anzusehen, da über Hydratationsvorgänge Feuchtigkeit in der Haut gebunden werden kann.

Aminosäuren mit besonders vorteilhafter kosmetischer bzw. dermatologischer Wirkung sind Glycin, Alanin, Valin, Leucin, Isoleucin, Phenylalanin, Tyrosin, Prolin, Hydroxyprolin, Serin, Threonin, Cystein, Methionin, Tryptophan, Arginin.

(3) Kosmetisch und dermatologisch relevante α-Hydroxycarbonsäuren, α-Ketocarbonsäuren und β-Hydroxycarbonsäuren und insbesondere deren Salze, wobei die Kationen vorteilhaft gewählt werden können aus der Gruppe Ammonium,- Alkylammonium,-Alkalimetall-, Erdalkalimetall,- Magnesium-, Eisen- bzw. Zinkionen.
α-Hydroxycarbonsäuren, welche kosmetisch oder dermatologisch relevant sind, folgen der allgemeinen Formel

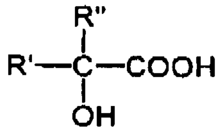

β-Hydroxycarbonsäuren, welche kosmetisch oder dermatologisch relevant sind, folgen der allgemeinen Formel

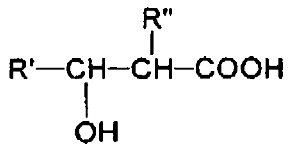

bzw.

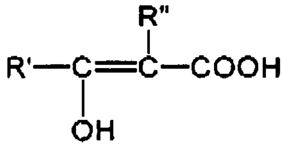

α-Ketocarbonsäuren, welche kosmetisch oder dermatologisch relevant sind, folgen der allgemeinen Formel

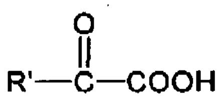

wobei jeweils R' und R" unabhängig voneinander gewählt werden aus den folgenden
Gruppen (a) bis (e)

(a) H-,
(b) verzweigtes oder unverzweigtes $C_{1-25}$ -Alkyl-,
(c) mit einer oder mehreren Carboxylgruppen und/oder Hydroxygruppen und/oder Aldehydgruppen und/oder Oxogruppen (Ketogruppen) substituiertes verzweigtes oder unverzweigtes $C_{1-25}$ -Alkyl-
(d) Phenyl-,
(e) mit einer oder mehreren Carboxylgruppen und/oder Hydroxygruppen und/oder verzweigten und/oder unverzweigten $C_{1-25}$-Alkylgruppen substituiertes Phenyl-, oder wobei das α-Kohlenstoffatom und das β-Kohlenstoffatom der β-Hydroxycarbonsäure mit R' und R" zusammen eine
(f) unsubstituierte Cycloalkylgruppe oder Arylgruppe mit 3 bis 7 Ringatomen oder eine
(g) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe oder Arylgruppe mit 3 bis 7 Ringatomen ausbilden
und wobei die α-Hydroxycarbonsäuren oder die β-Hydroxycarbonsäuren oder die α-Ketocarbonsäuren gegebenenfalls in Form ihrer physiologisch verträglichen Salze vorliegen können.
Die erfindungsgemäß verwendeten α-Hydroxycarbonsäuren, β-Hydroxycarbonsäuren und α-Ketocarbonsäuren werden vorteilhaft gewählt aus folgenden Substanzklassen, wobei diese auch stellvertretend für ihre Salze bzw. Anionen aufgeführt werden:
Salicylsäure (auch 2-Hydroxybenzoesäure, Spirsäure), welche durch die Struktur

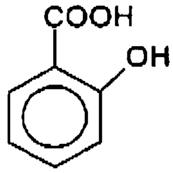

gekennzeichnet ist. Bekanntermaßen wirkt Salicylsäure antibakteriell und keratolytisch und ist Bestandteil mancher kosmetischen oder pharmazeutischen Zubereitungen.
Die erfindungsgemäß verwendeten α-Hydroxycarbonsäuren werden vorteilhaft gewählt aus den folgenden Substanzklassen (h) bis (k):
(h) α-Hydroxyfettsäuren, wobei diese wiederum besonders vorteilhaft aus der Gruppe der $C_{10-18}$ -Alkylcarbonsäuren gewählt werden,
(i) α-Hydroxyzuckersäuren und aliphatische α-Hydroxyfruchtsäuren,
(j) unsubstituierte aromatische α-Hydroxycarbonsäuren (z.B. Mandelsäure) bzw.
(k) substituierte aromatische α-Hydroxycarbonsäuren.

Die unter Punkt (h) fallenden α-Hydroxyfettsäuren werden besonders vorteilhaft gewählt aus der Gruppe

- α-Hydroxycarbonsäuren, gemäß der Formel

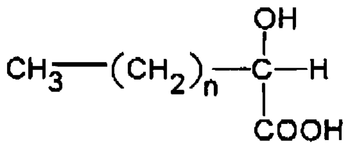

und/oder
- α-Hydroxy-isocarbonsäuren, gemäß der Formel

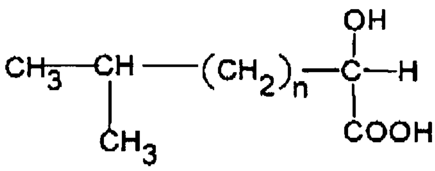

und/oder
- α-Hydroxy-anteisocarbonsäuren, gemäß der Formel

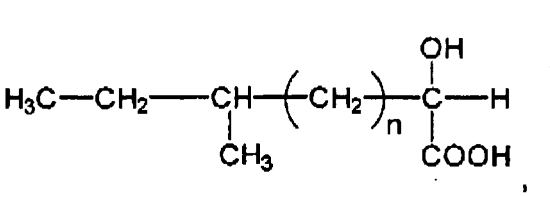

wobei n jeweils eine Zahl von 7 bis 31 darstellt.
Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.
Die unter Punkt (i) fallenden α-Hydroxyzuckersäuren werden besonders vorteilhaft gewählt aus der Gruppe der

- Aldonsäuren, z.B. Gluconsäure, Galactonsäure

- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure

Die unter Punkt (i) fallenden aliphatischen α-Hydroxyfruchtsäuren werden besonders vorteilhaft gewählt aus der Gruppe Äpfelsäure, Milchsäure, Citronensäure, Weinsäure.

Äpfelsäure (Hydroxybernsteinsäure) ist durch folgende chemische Struktur gekennzeichnet:

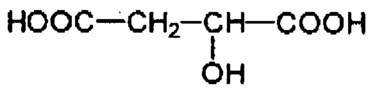

Milchsäure (2-Hydroxypropansäure) ist durch folgende chemische Struktur gekennzeichnet:

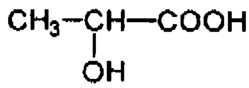

Citronensäure (2-Hydroxy-1,2,3-propantricarbonsäure) ist durch folgende chemische Struktur gekennzeichnet:

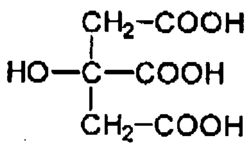

Bekanntermaßen wird Citronensäure zur Pufferung kosmetischer und/oder dermatologischer Zubereitungen, aber auch als Synergist für Antioxidantien in der Haut- und Haarkosmetik verwendet.

Weinsäure (Dihydroxybernsteinsäure) ist durch folgende chemische Struktur gekennzeichnet:

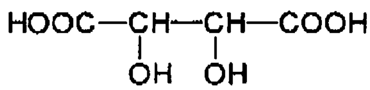

Bevorzugte α-Ketocarbonsäure ist die Brenztraubensäure (α-Oxopropansäure). Sie zeichnet sich durch folgende Struktur aus:

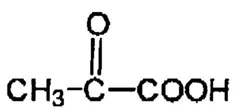

Die Höchstmenge der einzusetztenden Elektrolyte ist letztendlich abhängig von deren Löslichlichkeit in der wäßrigen Phase. Grundsetzlich setzt die erfindungsgemäße Lehre aber keine Höchstmengen als Schranke, da es gegebenenfalls ja sogar vorteilhaft sein mag, aus welchen Gründen auch immer, einen über die Löslichkeit eines Elektrolytes hinausgehende zusätzliche Menge dieses Elektrolytes, beispielsweise als ungelösten Festkörper, in eine kosmetische oder dermatologische Zubereitung einzuarbeiten.

**Ölphase**

Die Ölphase der erfindungsgemäßen W/O-Emulsionen wird vorteilhaft gewählt aus der Gruppe der Lecithine

und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl und dergleichen mehr.

Vorteilhaft werden die erfindungsgemäßen Öle ebenfalls gewählt aus der Gruppe Vaseline (Petrolatum), Paraffinöl und Polyolefine. Unter den Polyolefinen sind Polydecene die bevorzugten Substanzen.

Die Ölphase kann im Sinne der vorliegenden Erfindung ferner vorteilhaft gewählt werden aus der Gruppe der Ester aus gesättigten und/oder ungesättigten, ver-zweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

**Kosmetische oder dermatologische Hilfs- und Zusatzstoffe**

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander. Kombination.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-Oleyl-, y -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Zitronensäure, Milchsäure, Apfelsäure), Huminsäure, Gallensäure, Gallenextrakte, Bilirubin, Biliverdin,

EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch weitere Elektrolyte.

Letztere können beispielsweise gewählt werden aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraessigsäure und deren Salze und andere mehr. Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Mutatis mutandis gelten entsprechende Anforderungen an die Formulierung medizinischer Zubereitungen.

Die erfindungsgemäßen W/O-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Die dünnflüssigen kosmetischen oder dermatologischen Mittel gemäß der Erfindung können beispielsweise als aus Aerosolbehältern, Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Als Treibmittel für aus Aerosolbehältern versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden

können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescremes gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:

- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon,
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin,
- Derivate des 1,3,5-Triazins, vorzugsweise 2,4,6-Trianilino-(p-carbo-2'-ethyl-1'-hexyloxy)-1,3,5-Triazin.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Filtern zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Als weitere Bestandteile können verwendet werden:

- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiel 1**

| | |
|---|---|
| Isopropylstearat | 12,00 |
| Caprylylether | 8,00 |
| Cetearylisononanoat | 6,00 |
| Lameform® TGI[1] | 3,50 |
| Dehymuls® PGPH[2] | 3,50 |
| Eusolex® 232[3] | 3,50 |
| Glycerin | 3,00 |
| Magnesiumsulfat | 0,60 |
| Konservierungsmittel | q.s. |
| Parfum | q.s. |
| Wasser, demin. | ad 100 |

**Beispiel 2**

| | |
|---|---|
| $C_{12-15}$ Alkylbenzoat | 10,00 |
| Octyldodecanol | 7,00 |
| Eusolex® 232[3] | 5,50 |
| Lameform® TGI[1] | 5,00 |
| Capric/Caprylic Triglycerid | 5,00 |
| Natriumlactat (50 %) | 4,50 |
| Glycerin | 3,00 |
| Dehymuls® PGPH[2] | 2,50 |
| Milchsäure (90 %) | 1,50 |
| Aluminiumstearat | 0,20 |
| Konservierungsmittel | q.s. |
| Parfum | q.s. |
| Wasser, demin. | ad 100 |

**Beispiel 3**

| | |
|---|---|
| $C_{12-15}$ Alkylbenzoat | 10,00 |
| Octyldodecanol | 7,00 |
| Eusolex® 232[3] | 5,50 |
| Dehymuls® PGPH[2] | 5,00 |
| Capric/Caprylic Triglycerid | 5,00 |
| Natriumlactat (50 %) | 4,50 |

(fortgesetzt)

| | |
|---|---|
| Glycerin | 3,00 |
| Lameform® TGI[1] | 2,50 |
| Milchsäure (90 %) | 1,50 |
| Aluminiumstearat | 0,20 |
| Konservierungsmittel | q.s. |
| Parfum | q.s. |
| Wasser, demin. | ad 100 |

**Beispiel 4**

| | |
|---|---|
| Isopropylisostearat | 10,00 |
| $C_{12-15}$ Alkylbenzoat | 10,00 |
| Cetearylisononanoat | 6,00 |
| Butylenglykol | 5,00 |
| Lameform® TGI[1] | 3,50 |
| Dehymuls® PGPH[2] | 3,50 |
| Eusolex® 232[3] | 3,50 |
| Magnesiumsulfat | 0,60 |
| Konservierungsmittel | q.s. |
| Parfum | q.s. |
| Wasser, demin. | ad 100 |

**Beispiel 5**

| | |
|---|---|
| Caprylylether | 8,00 |
| Isopropylstearat | 7,00 |
| Cetearylisononanoat | 6,00 |
| Lameform® TGI[1] | 3,50 |
| Dehymuls® PGPH[2] | 3,50 |
| Eusolex® 232[3] | 3,50 |
| Glycerin | 3,00 |
| Uvinul® T150[4] | 3,00 |
| Magnesiumsulfat | 0,60 |
| Konservierungsmittel | q.s. |
| Parfum | q.s. |
| Wasser, demin. | ad 100 |

**Beispiel 6**

| | |
|---|---|
| Octyldodecanol | 7,00 |
| $C_{12-15}$ Alkylbenzoat | 6,00 |
| Lameform® TGI[1] | 5,00 |
| Capric/Caprylic Triglycerid | 5,00 |
| Uvinul® T150[4] | 5,00 |
| Eusolex® 232[3] | 5,00 |
| Natriumlactat (50 %) | 4,50 |
| Glycerin | 3,00 |
| Dehymuls® PGPH[2] | 2,50 |
| Milchsäure (90 %) | 1,50 |
| Aluminiumstearat | 0,20 |
| Konservierungsmittel | q.s. |
| Parfum | q.s. |
| Wasser, demin. | ad 100 |

**Beispiel 7**

| | |
|---|---|
| $C_{12-15}$ Alkylbenzoat | 10,00 |
| Cetearylisononanoat | 6,00 |
| Butylenglykol | 5,00 |
| Eusolex® 232[3] | 4,00 |
| Lameform® TGI[1] | 3,50 |
| Dehymuls® PGPH[2] | 3,50 |
| Isopropylisostearat | 3,00 |
| Natriumhydroxyd | 0,60 |
| Konservierungsmittel | q.s. |
| Parfum | q.s. |
| Wasser, demin. | ad 100 |

**Beispiel 8**

| | |
|---|---|
| Octyldodecanol | 7,00 |
| $C_{12-15}$ Alkylbenzoat | 6,00 |
| Dehymuls® PGPH[2] | 5,00 |
| Capric/Caprylic Triglycerid | 5,00 |
| Uvinul® T150[4] | 5,00 |

(fortgesetzt)

| | |
|---|---|
| Eusolex® 232[3] | 5,00 |
| Natriumlactat (50 %) | 4,50 |
| Glycerin | 3,00 |
| Lameform® TGI[1] | 2,50 |
| Milchsäure (90 %) | 1,50 |
| Aluminiumstearat | 0,20 |
| Konservierungsmittel | q.s. |
| Parfum | q.s. |
| Wasser, demin. | ad 100 |

**Beispiel 9**

| | |
|---|---|
| Caprylylether | 8,00 |
| Isopropylstearat | 7,00 |
| Cetearylisononanoat | 6,00 |
| Lameform® TGI[1] | 3,50 |
| Dehymuls® PGPH[2] | 3,50 |
| Eusolex® 232[3] | 3,50 |
| Glycerin | 3,00 |
| Uvinul® T150[4] | 3,00 |
| Parsol® 1789[5] | 2,00 |
| Eusolex® 6300[6] | 1,00 |
| Magnesiumsulfat | 0,60 |
| Konservierungsmittel | q.s. |
| Parfum | q.s. |
| Wasser, demin. | ad 100 |

**Beispiel 10**

| | |
|---|---|
| $C_{12-15}$ Alkylbenzoat | 10,00 |
| Octyldodecanol | 7,00 |
| Eusolex® 232[3] | 5,50 |
| Dehymuls® PGPH[2] | 5,00 |
| Capric/Caprylic Triglycerid | 5,00 |
| Natriumlactat (50 %) | 4,50 |
| Glycerin | 3,00 |
| Lameform® TGI[1] | 2,50 |

(fortgesetzt)

| | |
|---|---|
| Parsol® 1789[5] | 2,00 |
| Eusolex® 6300[6] | 1,00 |
| Milchsäure (90 %) | 1,50 |
| Aluminiumstearat | 0,20 |
| Konservierungsmittel | q.s. |
| Parfum | q.s. |
| Wasser, demin. | ad 100 |

**Beispiel 11**

| | |
|---|---|
| Caprylylether | 8,00 |
| Isopropylstearat | 7,00 |
| Cetearylisononanoat | 6,00 |
| Lameform® TGI[1] | 0,50 |
| Arlacel® P-135[7] | 3,50 |
| Eusolex® 232[3] | 3,50 |
| Glycerin | 3,00 |
| Uvinul® T150[4] | 3,00 |
| Magnesiumsulfat | 0,60 |
| Konservierungsmittel | q.s. |
| Parfum | q.s. |
| Wasser, demin. | ad 100 |

**Beispiel 12**

| | |
|---|---|
| Caprylylether | 8,00 |
| Isopropylstearat | 7,00 |
| Cetearylisononanoat | 6,00 |
| Arlacel® P-135[7] | 4,50 |
| Dehymuls® PGPH[2] | 0,50 |
| Eusolex® 232[3] | 3,50 |
| Glycerin | 3,00 |
| Uvinul® T150[4] | 3,00 |
| Parsol® 1789[5] | 2,00 |
| Eusolex® 6300[6] | 1,00 |
| Magnesiumsulfat | 0,60 |
| Konservierungsmittel | q.s. |

(fortgesetzt)

| | |
|---|---|
| Parfum | q.s. |
| Wasser, demin. | ad 100 |

[1] Triglycerindiisostearat
[2] Polyglycerinpolyhydroxystearat
[3] 2-Phenylbenzimidazol-5-sulfonsäure
[4] 4,4',4"-(1,3,5-Triazin-2,4,6-triyltriimino)-tris-benzoesäure-tris(2-ethylhexylester)
Butylmethoxydibenzoylmethan
[6] Methylbenzyliden Campher
[7] PEG-30 Polyhydroxystearat

**Patentansprüche**

**1.** Dünnflüssige kosmetische oder dermatologische Zubereitungen vom Typ Wasser-in-Öl, enthaltend

(1) eine Ölphase, enthaltend ein oder mehrere Öle, Fette und/oder Wachse,
(2) eine Wasserphase, wobei die Wasserphase einen oder mehrere Elektrolyte gelöst enthält, wobei die Ionenstärke der wässrigen Phase mindestens 0,075 mol/l beträgt und
(3) ein Emulgatorsystem, bestehend aus mindestens zwei Emulgatoren, welche gewählt werden aus der Gruppe Polyglyceryl-3-Diisostearat, Polyglyceryl-2-Polyhydroxystearat, PEG-30-Dipolyhydroxystearat,

**dadurch gekennzeichnet, dass** die Viskosität der Zubereitungen kleiner als 2.000 mPa·s ist.

**2.** Zubereitung nach Anspruch 1, **dadurch gekennzeichnet, dass** zusätzlich weitere kosmetische oder pharmazeutische Hilfs-, Zusatz- und/oder Wirkstoffe enthalten sind, die den Gehalt an (2) entsprechend vermindern.

**3.** Zubereitungen nach Anspruch 1, **dadurch gekennzeichnet, dass** die Viskosität dieser Zubereitungen kleiner als 1.500 mPa·s ist.

**4.** Zubereitungen nach Anspruch 1, **dadurch gekennzeichnet, dass** die Elektrolyte gewählt werden aus der Gruppe der wasserlöslichen UV-Filtersubstanzen, die an ihrem Molekülgerüst eine oder mehrere Sulfonsäuregruppen bzw. Sulfonatgruppen tragen.

**5.** Zubereitungen nach Anspruch 1, **dadurch gekennzeichnet, dass** die Elektrolyte gewählt werden aus der Gruppe der Aminosäuren und deren Salze.

**6.** Zubereitungen nach Anspruch 1, **dadurch gekennzeichnet, dass** die Elektrolyte gewählt werden aus der Gruppe der kosmetisch und dermatologisch relevanten α-Hydroxycarbonsäuren, α-Ketocarbonsäuren und β-Hydroxycarbonsäuren und deren Salze.

**7.** Verwendung von Zubereitungen nach Anspruch 1 als kosmetisches oder dermatologisches Vehikel zum Transport von Wirkstoffen in die Haut.

**Claims**

**1.** Low-viscosity cosmetic or dermatological preparations of the water-in-oil type, comprising

(1) an oil phase, comprising one or more oils, fats and/or waxes,
(2) a water phase, where the water phase comprises one or more electrolytes in dissolved form, where the ionic strength of the aqueous phase is at least 0.075 mol/l and
(3) an emulsifier system consisting of at least two emulsifiers, which are selected from the group consisting of polyglycerol-3 diisostearate, polyglyceryl-2 polyhydroxystearate, PEG-30 dipolyhydroxystearate,

**characterized in that** the viscosity of the preparations is less than 2000 mPa·s.

2. Preparation according to Claim 1, **characterized in that** additionally further cosmetic or pharmaceutical auxiliaries, additives and/or active ingredients are present, which accordingly reduce the content of (2).

3. Preparations according to Claim 1, **characterized in that** the viscosity of these preparations is less than 1500 mPa·s.

4. Preparations according to Claim 1, **characterized in that** the electrolytes are selected from the group of water-soluble UV filter substances which carry one or more sulphonic acid groups or sulphonate groups on their molecular backbone.

5. Preparations according to Claim 1, **characterized in that** the electrolytes are selected from the group of amino acids and salts thereof.

6. Preparations according to Claim 1, **characterized in that** the electrolytes are selected from the group of cosmetically and dermatologically relevant α-hydroxycarboxylic acids, α-ketocarboxylic acids and β-hydroxycarboxylic acids and salts thereof.

7. Use of preparations according to Claim 1 as cosmetic or dermatological vehicle for transporting active ingredients into the skin.

## Revendications

1. Préparations cosmétiques ou dermatologiques liquides du type eau-dans-huile, contenant

   (1) une phase huileuse, contenant une ou plusieurs huiles, graisses et/ou cires,
   (2) une phase aqueuse, où la phase aqueuse contient un ou plusieurs électrolytes sous forme dissoute, la force ionique de la phase aqueuse étant d'au moins 0,075 mole/l et
   (3) un système d'émulsifiant, constitué par au moins deux émulsifiants, qui sont choisis dans le groupe formé par le diisostéarate de polyglycéryle-3, le polyhydroxystéarate de polyglycéryle-2, le dipolyhydroxystéarate de PEG-30,

   **caractérisées en ce que** la viscosité des préparations est inférieure à 2000 mPa.s.

2. Préparation selon la revendication 1, **caractérisée en ce qu'**elle contient en outre d'autres adjuvants, additifs et/ou substances actives cosmétiques ou pharmaceutiques, qui diminuent de manière correspondante la teneur en (2).

3. Préparations selon la revendication 1, **caractérisées en ce que** la viscosité de ces préparations est inférieure à 1500 mPa.s.

4. Préparations selon la revendication 1, **caractérisées en ce que** les électrolytes sont choisis dans le groupe des substances filtre des UV solubles dans l'eau, qui portent sur leur structure moléculaire un ou plusieurs groupes acide sulfonique ou groupes sulfonate.

5. Préparations selon la revendication 1, **caractérisées en ce que** les électrolytes sont choisis dans le groupe des aminoacides et leurs sels.

6. Préparations selon la revendication 1, **caractérisées en ce que** les électrolytes sont choisis dans le groupe des acides α-hydroxycarboxyliques, α-cétocarboxyliques et β-hydroxycarboxyliques présentant une pertinence cosmétique et dermatologique et leurs sels.

7. Utilisation des préparations selon la revendication 1 comme véhicule cosmétique ou dermatologique pour le transport de substances actives dans la peau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5162378 A
- WO 9817232 A
- EP 834306 A
- DE 4126969
- DE 4243119
- DE 4420516
- DE 4409569
- DE 4409569 C1
- DE 4420516 A1

### In der Beschreibung aufgeführte Nicht-Patentliteratur

- *Seife, Öle, Fette, Wachse,* vol. 110 (15), 427 ff